# EUROPEAN PATENT APPLICATION

(11) **EP 2 258 470 A1**
(43) Date of publication of application: **08.12.2010**
(21) Application number: 09723492.6
(22) Date of filing: 19.01.2009
(51) Int. Cl.: B01J 20/22, G01N 30/88, G01N 33/543

(54) **METHOD FOR PRODUCING AFFINITY PARTICLES, AFFINITY PARTICLES, AND SEPARATION METHOD**

(30) Priority: 19.03.2008 JP 2008070750
(71) Applicant: Shiseido Company, Ltd., Chuo-ku Tokyo 104-8010 (JP)
(72) Inventor: MAENO, Katsuyuki, Yokohama-shi Kanagawa 224-8558 (JP)
(74) Representative: Hart-Davis, Jason
(86) International application number: PCT/JP2009/050686
(87) International publication number: WO 2009/116309

(57) **Abstract**

A method of manufacturing an affinity particle includes a step of reacting a particle having a reactive functional group on a surface thereof with a ligand having a functional group having a reactivity with the reactive functional group to bond the ligand to the particle, and a step of reacting the particle to which the ligand is bonded with a surface modifying agent having a functional group represented by a general formula of: 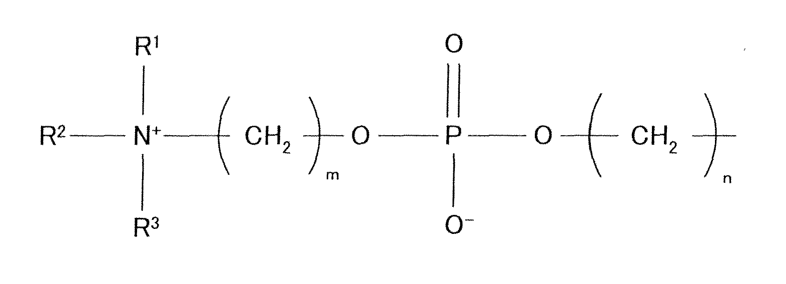 (wherein each of R¹, R², and R³ is independently an alkyl group whose carbon number is 1 or more and 6 or less, m is an integer of 2 or more and 6 or less, and n is 1 or 2.) and a functional group having a reactivity with the reactive functional group to bond the surface modifying agent to the particle to which the ligand is bonded.

## Description

### TECHNICAL FIELD

The present invention relates to a method of manufacturing an affinity particle, an affinity particle, and a separation method.

### BACKGROUND ART

Conventionally, column chromatography has been used for a method for separating or purifying a biological substance. However, a problem is that it is necessary to use various kinds of columns in order to obtain a target substance and so efficiency of purification is low. Furthermore, another problem is that it is necessary to confirm whether or not a target substance is included in fractionated components and a great amount of time is required for purification. Moreover, another problem is that a loss during purification is large and a great amount of a sample is needed.

Meanwhile, it has been known that an affinity particle has been used for separating or purifying a biological substance. Because a ligand is supported on a surface of an affinity particle, it is possible to selectively capture a target substance which is bound to a ligand specifically, but a problem is that a substance other than a target substance is adsorbed, which reduces an efficiency of capturing a target substance.

Herein, an affinity particle is known which has a phosphorylcholine group that covalently bonds to a surface of an organic particle or inorganic particle (see patent documents 1 and 2).

However, a part other than a ligand and a phosphorylcholine group is formed on a surface of an affinity particle in a manufacturing method disclosed in any of patent documents 1 and 2, and hence, a problem is that an efficiency of capturing a target substance is reduced or adsorption of a substance other than a target substance is increased.
Patent document 1: Japanese Patent Application Publication No. 2006-7203
Patent document 2: Japanese Patent Application Publication No. 2006-7204

### DISCLOSURE OF THE INVENTION

### PROBLEMS TO BE SOLVED BY THE INVENTION

The present invention aims at providing a method of manufacturing an affinity particle capable of being excellent in an efficiency of capturing a target substance and suppressing adsorption of a substance other than a target substance, an affinity particle manufactured by using the method of manufacturing an affinity particle, and a separation method using the affinity particle, while a problem possessed by a conventional technique as described above is taken into consideration.

### MEANS FOR SOLVING THE PROBLEM

The invention as recited in claim 1 is a method of manufacturing an affinity particle characterized by including a step of reacting a particle having a reactive functional group on a surface thereof with a ligand having a functional group having a reactivity with the reactive functional group to bond the ligand to the particle, and a step of reacting the particle to which the ligand is bonded with a surface modifying agent having a functional group represented by a general formula of: 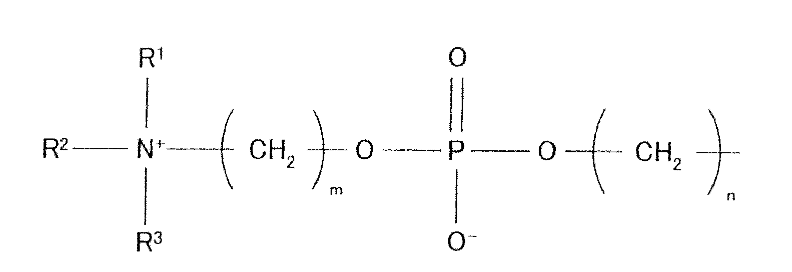 (wherein each of R¹, R², and R³ is independently an alkyl group whose carbon number is 1 or more and 6 or less, m is an integer of 2 or more and 6 or less, and n is 1 or 2.) and a functional group having a reactivity with the reactive functional group to bond the surface modifying agent to the particle to which the ligand is bonded.

The invention as recited in claim 2 is a method of manufacturing an affinity particle as recited in claim 1 wherein the reactive functional group is at least one of an amino group, a hydroxyl group, an aldehyde group, and a carboxyl group.

The invention as recited in claim 3 is an affinity particle manufactured by using a method of manufacturing an affinity particle as recited in claim 1.

The invention as recited in claim 4 is a separation method using an affinity particle as recited in claim 3 to separate a target substance.

### ADVANTAGEOUS EFFECT OF THE INVENTION

According to the present invention, it is possible to provide a method of manufacturing an affinity particle capable of being excellent in an efficiency of capturing a target substance and suppressing adsorption of a substance other than a target substance, an affinity particle manufactured by using the method of manufacturing an affinity particle, and a separation method using the affinity particle.

### BRIEF DESCRIPTION OF THE DRAWINGS

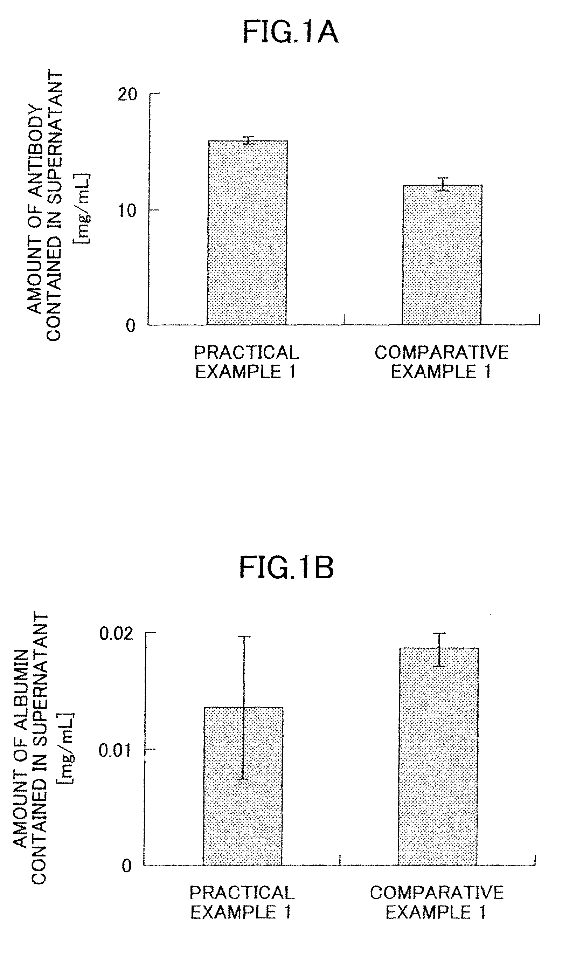

FIG. 1A is a diagram illustrating an amount of an antibody contained in a supernatant in practical example 1 and comparative example 1.
FIG. 1B is a diagram illustrating an amount of albumin contained in a supernatant in practical example 1 and comparative example 1.

### BEST MODE FOR CARRYING OUT THE INVENTION

Next, the best mode for carrying out the present invention will be described in conjunction with the drawings.

A method of manufacturing an affinity particle according to the present invention includes a step of reacting a particle having a reactive functional group on a surface thereof with a ligand having a functional group having a reactivity with the reactive functional group to bond the ligand to the particle, and a step of reacting the particle to which the ligand is bonded with a surface modifying agent having a functional group represented by general formula (1) of: 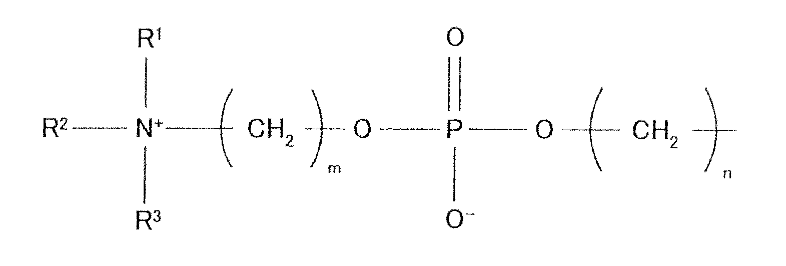 (wherein each of R¹, R², and R³ is independently an alkyl group whose carbon number is 1 - 6, m is an integer of 2 - 6, and n is 1 or 2.) and a functional group having a reactivity with the reactive functional group to bond the surface modifying agent to the particle to which the ligand is bonded. Thereby, it is possible to introduce the ligand and a phosphorylcholine-like group onto a surface of the particle at high densities. As a result, it is possible to obtain an affinity particle excellent in an efficiency of capturing a target substance and capable of suppressing adsorption of a substance other than the target substance.

In the present invention, a reactive functional group held on a particle surface is not particularly limited and at least one of an amino group, a hydroxyl group, an aldehyde group, and a carboxyl group is preferable.

A particle having such a reactive functional group on a surface thereof may either be an organic particle or an inorganic particle, and preferably, has an average particle diameter of 20 nm - 500 µm.

A material constituting an organic particle is not particularly limited and it is possible to provide a homopolymer or copolymer obtained by polymerizing a monomer(s) such as styrene, glycidyl methacrylate, (meth)acrylic acid, an N-alkylacrylamide, an alkyl (meth)acrylate, an aminoalkyl (meth)acrylate, and/or an hydroxyalkyl (meth)acrylate, or the like. Among these, preferable is an acrylic acid - N-isopropylacrylamide - methylene bis(acrylamide) copolymer, a 2-hydroxyethyl methacrylate - styrene - divinylbenzene copolymer, a 2-aminoethyl methacrylate - N-isopropylacrylamide - methylene bis(acrylamide) copolymer, or the like. It is possible to synthesize such an organic particle by means of emulsion polymerization, suspension polymerization, or the like.

For a material constituting an organic material other than these ones, it is also possible to provide an agarose, a sepharose, or the like.

A material constituting an inorganic particle is not particularly limited and it is possible to provide a talc, a kaolin, a mica, sericite (SERICITE), muscovite, phlogopite, a synthetic mica, lepidolite, biotite, vermiculite, magnesium carbonate, calcium carbonate, aluminum silicate, barium silicate, calcium silicate, magnesium silicate, strontium silicate, a metal salt of tungstic acid, magnesium, silica, a zeolite, barium sulfate, a calcined calcium sulfate (calcined gypsum), calcium phosphate, fluoroapatite, hydroxylapatite, a ceramic powder, a metal soap, (for example, zinc myristate, calcium palmitate, or aluminum stearate), boron nitride, a cerium oxide, a gold colloid, or the like. Among these, preferable is silica, a titanium oxide, zinc oxide, an alumina, an iron oxide, a talc, a mica, sericite, a gold colloid, or the like.

Additionally, a reactive functional group may be introduced by modifying a particle surface for a particle having a reactive functional group on a surface thereof.

In a method for introducing an amino group onto a particle surface, it is possible to provide a plasma treatment, a method of reacting a surface modifying agent, or a silicone gas-phase treatment.

In regard to a plasma treatment, an amino group is introduced onto a particle surface by means of a low-temperature plasma in nitrogen gas atmosphere (for example, see Surface and Coatings Technology, 116-119, 802-807 (1999), Colloids and Surfaces A: Physicochem. Eng. Aspects, 195, 81-95 (2001), Macromol. Chem. Phys., 200, 989-996 (1999)). Specifically, after a particle is contained in a reaction vessel and the content of the reaction vessel is evacuated by a vacuum pump, a nitrogen gas is introduced thereinto and glow discharge is conducted. Then, a plasma-treated material may be mechanically formed into a particle(s) to manufacture a particle having an amino group on a surface thereof.

In regard to a method of reacting a surface modifying agent, a surface modifying agent such as an alkoxysilane, chlorosilane, or silazane having an amino group is used to introduce an amino group onto a particle surface having a silanol group, an alkoxysilyl group or the like. Specifically, after a particle is first dipped in a mixed liquid of water/2-propanol and 3-aminopropyltrimethoxysilane is added thereto, a reaction is conducted by heating at 100 °C for 6 hours. After cooling is then conducted to room temperature, washing with methanol and drying are conducted. For a particle, it is possible to provide an organic particle of a 3-trimethoxysilylpropyl methacrylate - methyl methacrylate - divinylbenzene copolymer or the like; or an inorganic particle of silica, a glass, an alumina, a talc, a clay, a mica, an asbestos, a titanium oxide, zinc oxide, an iron oxide, or the like; or the like.

In regard to a silicone gas-phase treatment, after 1,3,5,7-tetramethylcyclotetrasiloxane is used to introduce a hydrosilyl group onto a particle surface, a monomer having an amino group is reacted to introduce an amino group onto a particle surface (For example, see Japanese Examined Patent Application Publication No. H1-54379, Japanese Examined Patent Application Publication No. H1-54380, and Japanese Examined Patent Application Publication No. H1-54381). Specifically, a particle(s) and 1,3,5,7-tetramethylcyclotetrasiloxane are first put in a desiccator and degassing is conducted by an aspirator. After a reaction is then conducted at 80 °C for 16 hours, a particle(s) is/are removed and dried at 120 °C. Furthermore, after an obtained particle(s) is/are dispersed in ethanol and allylamine is added thereto, a solution of chloroplatinic acid in ethanol is added and stirring is conducted at 60 °C for 2 hours. After the reaction is completed, filtration, washing with ethanol, and drying under reduced pressure are conducted. For a particle, it is possible to provide an organic particle of a styrene - divinylbenzene copolymer or the like; an inorganic particle of a mica, a talc, a kaolin, an alumina, a titanium oxide, zinc oxide, an iron oxide, or the like; or the like. A monomer having an amino group is not limited to allylamine, and a vinyl monomer or acryl monomer having an amino group or the like is only needed. Furthermore, an amino group may be protected by a butoxycarbonyl group, a benzyloxycarbonyl group, or the like. Moreover, a monomer having a functional group capable of introducing an amino group, for example, by means of reaction with a diamine, such as an epoxy group, may be used instead of a monomer having an amino group.

For a method of introducing an aldehyde group onto a particle surface, it is possible to provide a method of reacting glutaraldehyde with a particle having an amino group on a surface thereof.

For a method of introducing a carboxyl group onto a particle surface, it is possible to provide a method of reacting a surface modifying agent, a silicone gas-phase treatment, or the like.

In regard to a method of reacting a surface modifying agent, a surface modifying agent such as an alkoxysilane, chlorosilane, or silazane having a carboxyl group is used to introduce a carboxyl group onto a particle surface having a silanol group, an alkoxysilyl group, or the like. Specifically, while triethoxysilylpropylsuccinic anhydride is first dissolved in N,N-dimethylformamide and a distilled water and 4-dimethylaminopyridine are added thereto, stirring is conducted at room temperature for 16 hours to synthesize a silane coupling agent having a carboxyl group. After a particle is then dipped in a mixed liquid of water/2-propanol and a silane coupling agent having a carboxyl group is added thereto, a reaction is conducted by heating at 100 °C for 6 hours. Furthermore, after cooling is conducted to room temperature, washing with methanol and drying are conducted. For a particle, it is possible to provide an organic particle of 3-trimethoxysilylpropyl methacrylate - methyl methacrylate - divinylbenzene copolymer or the like; an inorganic particle of silica, a glass, an alumina, a talc, a clay, a mica, an asbestos, a titanium oxide, zinc oxide, an iron oxide, or the like; or the like.

In regard to a silicone gas-phase treatment, after 1,3,5,7-tetramethylcyclotetrasiloxane is used to introduce a hydrosilyl group onto a particle surface, a monomer having a carboxyl group is reacted to introduce a carboxyl group onto a particle surface (for example, see Japanese Examined Patent Application Publication No. H1-54379, Japanese Examined Patent Application Publication No. H1-54380, and Japanese Examined Patent Application Publication No. H1-54381). Specifically, a particle(s) and 1,3,5,7-tetramethylcyclotetrasiloxane are first put in a desiccator and degassing is conducted by an aspirator. After a reaction is then conducted at 80 °C for 16 hours, a particle(s) is/are removed and dried at 120 °C. Furthermore, after an obtained particle(s) is/are dispersed in ethanol and allylcarboxylic acid is added thereto, a solution of chloroplatinic acid in ethanol is added and stirring is conducted at 60 °C for 2 hours. After the reaction is completed, filtration, washing with ethanol, and drying under reduced pressure are conducted. For a particle, it is possible to provide an organic particle of a styrene - divinylbenzene copolymer or the like; an inorganic particle of a mica, a talc, a kaolin, an alumina, a titanium oxide, zinc oxide, an iron oxide, or the like; or the like. A monomer having a carboxyl group is not limited to allylcarboxylic acid and a vinyl monomer or acryl monomer having a carboxyl group or the like is only needed.

In the present invention, a functional group having a reactivity with a reactive functional group held by a ligand is not particularly limited. Specifically, it is possible to provide a carboxyl group, an aldehyde group, or the like as a functional group having a reactivity with an amino group or a hydroxyl group, wherein a caroboxyl group is preferable from the viewpoint of a high reactivity. Furthermore, it is possible to provide an amino group, a hydroxyl group, or the like, as a functional group having a reactivity with an aldehyde group or a carboxyl group, wherein an amino group is preferable from the viewpoint of a high reactivity.

Furthermore, in regard to a ligand, a functional group having a reactivity with a reactive functional group is preferably bonded via a spacer. A spacer is not particularly limited and it is possible to provide a methylene group, an oxyethylene group, an alkylene group having one or more amino groups, or the etc.

A ligand is not particularly limited and it is possible to provide, any kind of antibody such as IgG, IgM, IgA, IgD, IgE, or IgY; an antigen such as a protein or a polysaccharide; an enzyme such as glutathione-S-transferase; a substrate such as glutathione; a receptor such as a hormone receptor or a cytokine receptor; a peptide, a DNA, an RNA, an aptamer, protein A, protein G, avidin, or a biotin; a chelate compound such as nitrilotriacetic acid; any kind of metal ion such as Ni²⁺, Co²⁺, Cu²⁺, Zn²⁺, or Fe³⁺; or the like.

When a ligand is a protein, a particle having an aldehyde group on a surface thereof and an amino group of a protein are condensed by means of a general reaction to form an imino bond. Specifically, after a particle and a surface modifying agent are left in methanol at room temperature for 6 hours, sodium cyanotrihydroborate is added thereto at 0 °C and heating and stirring are conducted overnight. Additionally, it is possible to use a protic solvent such as water, ethanol, or 2-propanol, for a reaction solvent, other than methanol, and an introdcution rate tends to be high in the case where methanol is used.

Furthermore, a particle having a caroboxyl group on a surface thereof and an amino group of a protein are condensed by means of a general reaction to form an amide bond. Specifically, after a particle is dipped in a solution of N-hydroxysuccinimide or 1-ethyl-3-(3-dimethylaminopropyl)carbodiimide to active-esterify a carboxyl group of a particle, a protein is added thereto.

Moreover, a particle having an amino group on a surface thereof and an amino group of a protein are condensed through glutaraldehyde by means of a general reaction to form an imino bond. Specifically, after glutaraldehyde is reacted with a particle (or a protein), a protein (or a particle) is reacted therewith.

Furthermore, a particle having a hydroxyl group on a surface thereof and a carboxyl group of a protein are condensed by means of a general reaction to form an ester bond. Specifically, after cyanogen bromide is used to activate a hydroxyl group of a particle, a protein is added thereto.

In the present invention, a molecular weight of a surface modifying agent for reacting with a particle to which a ligand is bonded is preferably 255 - 549 and more preferably 255 - 283. Thereby, it is possible to introduce a phosphorylcholine group onto a particle surface at a higher density.

Furthermore, a functional group having a reactivity with a reactive functional group held by a surface modifying agent is not particularly limited. Specifically, it is possible to provide a carboxyl group, an aldehyde group, or the like, as a functional group having a reactivity with an amino group or a hydroxyl group, wherein a carboxyl group is preferable from the viewpoint of a high reactivity. Moreover, it is possible to provide an amino group, a hydroxyl group, or the like, as a functional group having a reactivity with an aldehyde group or a carboxyl group, wherein an amino group is preferable from the viewpoint of a high reactivity.

Furthermore, in regard to a surface modifying agent, a functional group having a reactivity with a reactive functional group is preferably bonded to a phosphorylcholine-like group via a spacer. A spacer is not particularly limited and it is possible to provide a methylene group, an oxyethylene group, an alkylene group having one or more amino groups, or the like.

Additionally, a functional group having a reactivity with a reactive functional group held by a surface modifying agent may be identical to or different from a reactive functional group held by a ligand.

A surface modifying agent will be specifically described below.

### (A surface modifying agent having an amino group)

While a surface modifying agent having an amino group is not particularly limited, it is possible to provide, for example, a compound disclosed in Japanese Patent Application Publication No. 2006-7203 or Japanese Patent Application Publication No. 2006-7204 or the like, and among these, preferable is a compound represented by general formula (2): 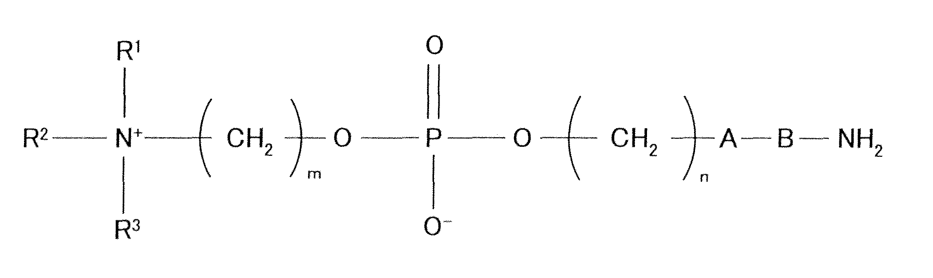 (wherein each of R¹, R², and R³ is independently an alkyl group whose carbon number is 1 - 6, A is an amino group, an ester bond, or an amide bond, B is an alkylene group whose carbon number is 1 - 3, a polyoxyethylene group whose carbon number is 1 - 3, or an arylene group, m is an integer of 2 - 6, and n is 1 or 2.).

When A is an imino group, it is possible to synthesize a compound represented by general formula (2) by, for example, oxidizing glycerophosphorylcholine with periodic acid to synthesize a phosphorylcholine derivative having an aldehyde group and subsequently conducting condensation with a compound having an amino group. Furthermore, when A is an amide bond or an ester bond, it is possible to synthesize a compound represented by general formula (2) by, for example, a method of oxidizing glycerophosphorylcholine with periodic acid and ruthenium trichloride to synthesize a phosphorylcholine derivative having a carboxyl group and subsequently conducting condensation with a compound having an amino group or a hydroxyl group.

When A is an amide bond or an ester bond, it is possible to synthesize a compound represented by general formula (2) by, for example, oxidizing glycerophosphorylcholine with permanganic acid and hydrochloric acid to synthesize a phosphorylcholine derivative having a carboxyl group and subsequently conducting condensation with a compound having an amino group or a hydroxyl group.

Next, a method of manufacturing a compound represented by general formula (2) will be described specifically.

### (An example of manufacturing of a surface modifying agent A)

After L-α-glycerophosphorylcholine (commercial product) represented by structural formula (1): 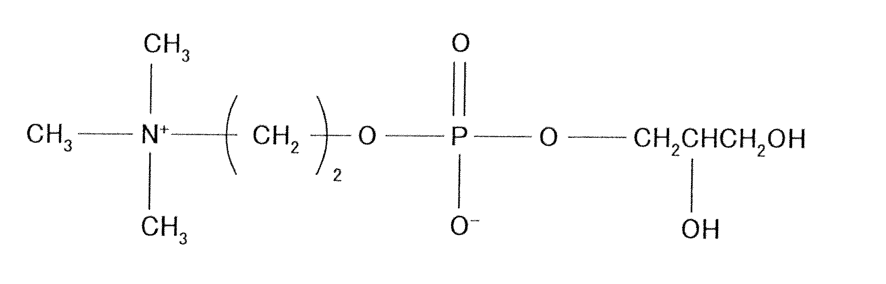 is first dissolved in a distilled water and cooling in an ice-water bath is conducted, sodium periodate is added thereto and stirring is conducted for 5 hours. After concentration under reduced pressure and drying under reduced pressure are further conducted, an extraction with methanol is conducted to obtain a phosphorylcholine derivative represented by structural formula (2): 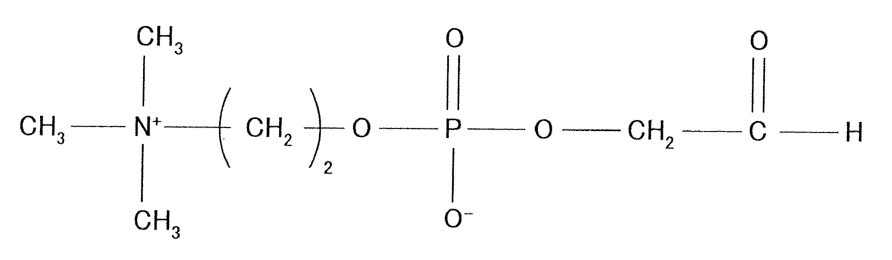 After a phosphorylcholine derivative represented by structural formula (2) is then dissolved in methanol, ethylenediamine is added thereto and stirring is conducted at room temperature. After cooling is further conducted in an ice-water bath, sodium cyanotrihydroborate is added thereto and warming to room temperature and stirring for 16 hours are conducted. Herein, dried nitrogen continues to flow through a reaction vessel. After a precipitate is then eliminated by filtration, concentration under reduced pressure and drying under reduced pressure are conducted to obtain a surface modifying agent A represented by structural formula (3):

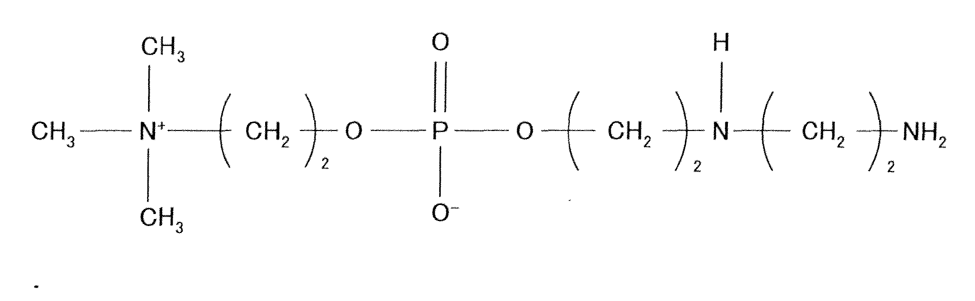

### (Example 1 of manufacturing of a surface modifying agent B)

After an aqueous solution of L-α-glycerophosphorylcholine is first cooled in an ice-water bath, sodium periodate and ruthenium trichloride are added thereto and stirring is conducted for 3 hours. After methanol is then added thereto and stirring is further conducted for 30 minutes, a precipitate is eliminated by filtration and concentration under reduced pressure and drying under reduced pressure are conducted to obtain a phosphorylcholine derivative represented by structural formula (4):

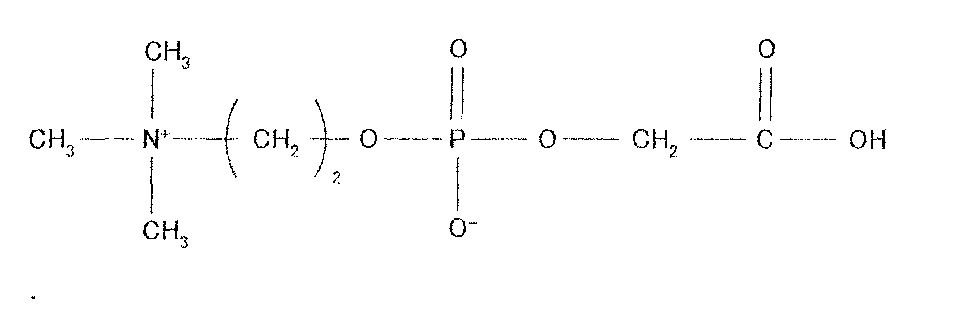

After ethylenediamine is then added to a solution of a phosphorylcholine derivative represented by structural formula (4) in methanol, a triazine-based dehydration and condensation agent (DMT-MM) is added thereto and stirring is conducted for 3 hours. Furthermore, a precipitate is eliminated by filtration and concentration under reduced pressure and drying under reduced pressure are conducted to obtain a surface modifying agent B represented by structural formula (5):

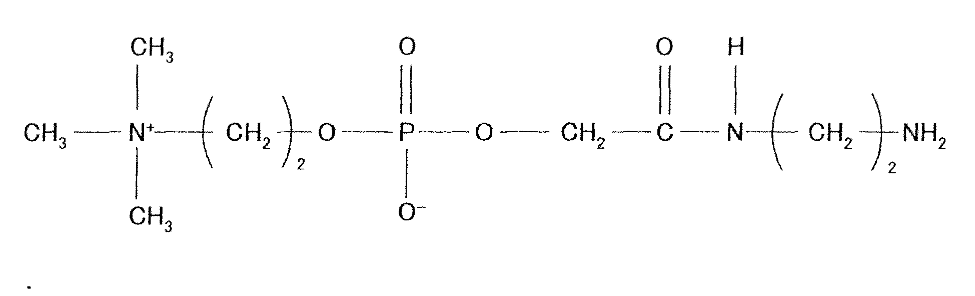

### (Example 2 of manufacturing of a surface modifying agent B)

After L-α-glycerophosphorylcholine is first cooled in an ice-water bath and simultaneously dissolved in hydrochloric acid, potassium permanganate is added thereto and stirring is conducted for 3 hours. After methanol is then added thereto and stirring is further conducted for 30 minutes, a precipitate is eliminated by filtration and concentration under reduced pressure and drying under reduced pressure are conducted to obtain a phosphorylcholine derivative represented by structural formula (4).

After ethylenediamine is then added to a solution of a phosphorylcholine derivative represented by structural formula (4) in methanol, a triazine-based dehydration and condensation agent (DMT-MM) is added thereto and stirring is conducted for 3 hours. Furthermore, a precipitate is eliminated by filtration and concentration under reduced pressure and drying under reduced pressure are conducted to obtain a surface modifying agent B represented by structural formula (5).

A particle having a carboxyl group on a surface thereof and a surface modifying agent having an amino group are condensed by means of a general reaction to form an amide bond. Specifically, after a particle is dipped in a solution of N-hydroxysuccinimide or 1-ethyl-3-(3-dimethylaminopropyl)carbodiimide to active-esterify a carboxyl group of a particle, a surface modifying agent is added thereto.

A particle having an aldehyde group on a surface thereof and a surface modifying agent having an amino group are condensed by means of a general reaction to form an imino bond. Specifically, after a particle and a surface modifying agent are left in methanol at room temperature for 6 hours, sodium cyanotrihydroborate is added thereto at 0 °C and heating and stirring are conducted overnight. Additionally, it is possible to use a protic solvent such as water, ethanol, or 2-propanol, for a reaction solvent, other than methanol, and an introduction rate tends to be high in the case where methanol is used.

### (A surface modifying agent having a hydroxyl group)

A surface modifying agent having a hydroxyl group is not particularly limited and it is possible to provide, for example, L-α-glycerophosphorylcholine or the like. For a method of manufacturing a surface modifying agent having a hydroxyl group, it is possible to provide, for example, a method of reducing a phosphorylcholine derivative represented by structural formula (2) or phosphorylcholine derivative represented by structural formula (4) with sodium borohydride or the like.

A particle having a carboxyl group on a surface thereof and a surface modifying agent having a hydroxyl group are condensed by means of a general reaction to form an ester bond. Specifically, after a hydroxyl group of a surface modifying agent is activated by using cyanogen bromide, dipping of a particle is conducted.

A particle having an aldehyde group on a surface thereof and a surface modifying agent having a hydroxyl group are added by means of a general reaction to form an acetal bond. Specifically, after a particle and a surface modifying agent are left in methanol at room temperature for 6 hours, sodium cyanotrihydroborate is added at 0 °C and heating and stirring are conducted overnight. Additionally, it is possible to use a protic solvent such as water, ethanol, or 2-propanol, for a reaction solvent, other than methanol, and an introduction rate tends to be high in the case where methanol is used.

### (A surface modifying agent having an aldehyde group)

A surface modifying agent having an aldehyde group is not particularly limited and it is possible to provide, for example, a compound disclosed in Japanese Patent Application Publication No. 2006-11383 or the like.

A particle having a hydroxyl group on a surface thereof and a surface modifying agent having an aldehyde group are added by means of a general reaction to form an acetal bond. Specifically, after a particle and a surface modifying agent are left in methanol at room temperature for 6 hours, sodium cyanotrihydroborate is added at 0 °C and heating and stirring are conducted overnight. Additionally, it is possible to use a protic solvent such as water, ethanol, or 2-propanol, for a reaction solvent, other than methanol, and an introduction rate tends to be high in the case where methanol is used.

A particle having an amino group on a surface thereof and a surface modifying agent having an aldehyde group are condensed by means of a general reaction to form an imino bond. Specifically, after a particle and a surface modifying agent are left in methanol at room temperature for 6 hours, sodium cyanotrihydroborate is added at 0 °C and heating and stirring are conducted overnight. Additionally, it is possible to use a protic solvent such as water, ethanol, or 2-propanol, for a reaction solvent, other than methanol, and an introduction rate tends to be high in the case where methanol is used.

### (A surface modifying agent having a carboxyl group)

A surface modifying agent having a carboxyl group is not particularly limited and it is possible to provide, for example, a compound disclosed in Japanese Patent Application Publication No. 2006-11381 or the like.

A particle having a hydroxyl group on a surface thereof and a surface modifying agent having a carboxyl group are condensed by means of a general reaction to form an ester bond. Specifically, after cyanogen bromide is used to activate a hydroxyl group of a surface modifying agent, a particle is added thereto.

A particle having an amino group on a surface thereof and a surface modifying agent having a carboxyl group condenses the amino group and the carboxyl group by means of a general reaction to form an amide bond. Specifically, after a surface modifying agent is dipped in a solution of N-hydroxysuccinimide or 1-ethyl-3-(3-dimethylaminopropyl)carbodiimide to active-esterify a carboxyl group, dipping of a particle is conducted.

It is possible to manufacture an affinity particle according to the present invention as described above, and an affinity particle is dispersed in a liquid sample which contains a target substance to bond to a ligand specifically, whereby it is possible to capture a target substance selectively. Specifically, after an affinity particle is first dispersed in a liquid sample which contains a target substance and moderate shaking is conducted at 4 °C for 30 minutes, centrifugation at 15,000 rpm is conducted for 30 minutes and a supernatant is discarded. After 1 ml of a PBS solution is then added and moderate shaking is conducted, centrifugation at 15,000 rpm is conducted for 30 minutes and a supernatant is discarded. Such a washing operation is repeated three times. Furthermore, a captured target substance is recovered from an affinity particle. Specifically, 1 ml of an extraction buffer is added thereto and moderate shaking is conducted at 4 °C for 30 minutes, whereby a target substance is extracted from an affinity particle, while a supernatant is recovered. After 1 ml of an extraction buffer is then added thereto and moderate shaking is conducted, centrifugation at 15,000 rpm is conducted for 30 minutes and a supernatant is recovered. Such operations are repeated two times. Thereby, it is possible to separate a target substance.

Additionally, an affinity particle may be used as a packing material for an affinity column to separate a target substance.

### [Practical example 1]

After 1.5 mmol of 3-aminopropyltrimethoxysilane, 380 mL of methanol, 20 mL of ultrapure water, and 60 g of silica gel with an average particle diameter of 50 µm were put in a flask and refluxed at 70 °C overnight, filtration washing was conducted by using methanol and water to obtain silica particles having an amino group on the surface thereof. After 500 mL of 4% by weight solution of glutaraldehyde in water and 5 g of sodium cyanotrihydroborate were then added to 50 g of the obtained silica particle and reaction was conducted at room temperature for 5 hours, filtration washing was conducted by using a PBS (phosphate buffered saline) to obtain silica particles having an aldehyde group on the surface thereof. Furthermore, after 80 mL of a 10 mg/mL solution of protein A (ligand) in water and 0.8 g of sodium cyanotrihydroborate were added and reacted at room temperature for 1 day, filtration washing was conducted by using a PBS to obtain silica particles having a ligand and an aldehyde group on the surface thereof. After 600 mL of 0.5 M solution (pH 7.0) of a surface modifying agent having an amino group (surface modifying agent B) in water and 6 g of sodium cyanotrihydroborate were then added to conduct reaction at room temperature for 2 hours, filtration washing was conducted by using PBS to obtain silica particles (affinity particles) having a ligand and a phosphorylcholine group on the surface thereof.

### [Comparative example 1]

After 1.5 mmol of 3-aminopropyltrimethoxysilane, 1.5 mL of 0.15 M solution of a surface modifying agent represented by structural formula: 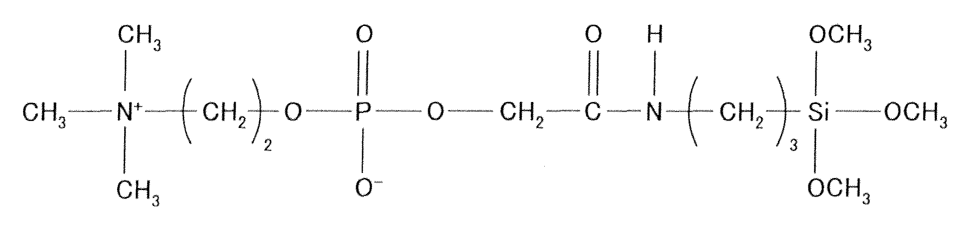 in methanol, 47.5 mL of methanol, 2.5 mL of distilled water, and 60 g of silica gel with an average particle diameter of 50 µm were put in a flask and refluxed at 70 °C overnight, filtration washing was conducted by using methanol and water to obtain silica particles having a phosphorylcholine group and an amino group on the surface thereof. After 500 mL of 4% by weight solution of glutaraldehyde in water and 5 g of sodium cyanotrihydroborate were then added to 50 g of the obtained silica particles and reaction was conducted at room temperature for 5 hours, filtration washing was conducted by using PBS to obtain silica particles having a phosphorylcholine group and an aldehyde group on the surface thereof. After 80 mL of 10 mg/mL solution of protein A (ligand) in water further and 0.8 g of sodium cyanotrihydroborate were added and reacted at room temperature for 1 day, filtration washing was conducted by using PBS to obtain silica particles having a phosphorylcholine group, a ligand, and an unreacted aldehyde group on the surface thereof. After 600 mL of 0.5 M solution (pH 7.0) of ethanolamine in water and 6 g of sodium cyanotrihydroborate were then added and reacted at room temperature for 2 hours, filtration washing was conducted by using PBS to obtain silica particles (affinity particles) having a phosphorylcholine group, a ligand, and a hydroxyl group on the surface thereof.

### [Evaluation method and evaluation results]

25 mL of the affinity particles and 2 mL of a 5 times diluted human serum were put in an Eppendorf tube and reacted at room temperature for 1 hour. After centrifugation (5,000 g) was then conducted to eliminate a supernatant, centrifugation (5,000 g) was conducted 5 times while PBS was used. After 500 µL of 0.2 M of Gly-HCl buffer (pH 2.5) was further added thereto and reaction was conducted at room temperature for 1 hour to extract an antibody, centrifugation (5,000 g) was conducted to obtain a supernatant. The quantities of the antibody (target substance) and albumin (impurity) contained in the obtained supernatant were quantified by using an ELISA method.

The evaluation results are illustrated in FIG. 1A and FIG. 1B. From FIG. 1A and FIG. 1B, it is found that the affinity particles in practical example 1 were excellent in an efficiency of capturing an antibody and suppressed adsorption of albumin. From this, it is considered that the ligands and the phosphorylcholine groups had been introduced onto the surface of the silica particle at a high density. On the other hand, it is considered that the amount of the introduced protein A decreased in regard to the affinity particles in comparative example 1, because the protein A was introduced after the phosphorylcholine groups were introduced, whereby, as a result, the efficiency of capturing the antibody decreased and adsorption of albumin increased.

The present international application claims the priority based on Japanese Patent Application No. 2008-70750 filed on March 19, 2008 and the entire content of Japanese Patent Application No. 2008-70750 is incorporated by reference in the present international application.

## Claims

1. A method of manufacturing an affinity particle comprising a step of reacting a particle having a reactive functional group on a surface thereof with a ligand having a functional group having a reactivity with the reactive functional group to bond the ligand to the particle, and a step of reacting the particle to which the ligand is bonded with a surface modifying agent having a functional group represented by a general formula of: 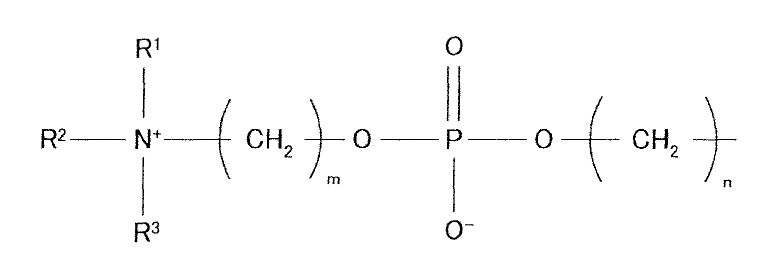 (wherein each of R¹, R², and R³ is independently an alkyl group whose carbon number is 1 or more and 6 or less, m is an integer of 2 or more and 6 or less, and n is 1 or 2.) and a functional group having a reactivity with the reactive functional group to bond the surface modifying agent to the particle to which the ligand is bonded.

2. The method of manufacturing an affinity particle as claimed in claim 1, wherein the reactive functional group is at least one of an amino group, a hydroxyl group, an aldehyde group, and a carboxyl group.

3. An affinity particle manufactured by using the method of manufacturing an affinity particle as
claimed in claim 1.

4. A separation method using the affinity particle as claimed in claim 3 to separate a target substance.
